# FASCICULE DE BREVET EUROPEEN

(11) **EP 3 337 443 B1**
(45) Date de publication et mention de la délivrance du brevet: **17.06.2020**
(21) Numéro de dépôt: 16760769.6
(22) Date de dépôt: 12.08.2016
(51) Int. Cl.: A61H 15/00, A61H 15/02

(54) **APPAREIL DE MASSAGE MUNI D'AU MOINS UNE COMPOSITION COSMETIQUE, DERMATOLOGIQUE ET/OU PHARMACEUTIQUE**
MASSAGEVORRICHTUNG MIT MINDESTENS EINER KOSMETISCHEN, DERMATOLOGISCHEN UND/ODER PHARMAZEUTISCHEN ZUSAMMENSETZUNG
MASSAGE DEVICE PROVIDED WITH AT LEAST ONE COSMETIC, DERMATOLOGICAL AND/OR PHARMACEUTICAL COMPOSITION

(30) Priorité: 18.08.2015 FR 1557795
(43) Date de publication de la demande: 27.06.2018
(73) Titulaire: SEB S.A., 69130 Ecully (FR)
(72) Inventeur: MANDICA, Franck, 69340 Francheville (FR)
(74) Mandataire: Bourrières, Patrice
(86) Numéro de dépôt international: PCT/FR2016/052075
(87) Numéro de publication internationale: WO 2017/029446

(56) Documents cités:
- EP-A1- 1 932 500
- EP-A1- 2 735 295
- EP-A1- 2 862 554
- EP-A1- 2 862 556
- GB-A- 2 495 973
- US-A1- 2014 163 443

## Description

### 1. Domaine de l'invention

L'invention concerne le domaine des appareils de traitement d'une surface corporelle externe. L'appareil selon l'invention permet, pour le moins, le massage de la surface corporelle externe afin de lui donner de la tonicité. L'appareil de massage selon l'invention trouvera son application chez des utilisateurs désireux de soigner leur esthétique en remodelant, raffermissant et rajeunissant une surface corporelle telle que la peau du visage.

### 2. Art antérieur

Il est connu des appareils de massage comprenant une tête de massage comprenant des moyens massants mobiles en rotation par rapport à la tête de massage et qui sont agencés de sorte à être en contact avec une surface corporelle externe d'un utilisateur, et un boitier muni de moyens d'entrainement actionnant les moyens massants. De tels appareils de massage sont connus des documents de brevet FR 3 012 034 et FR 3 012 035.

Toutefois, l'un des problèmes liés à ces appareils de massage est le fait que lorsque l'utilisateur souhaite combiner l'action d'une composition cosmétique, dermatologique et/ou pharmaceutique pour traiter un problème d'hydratation, de remodelage, d'anti-âge, d'acné, etc., en combinaison avec l'appareil de massage, celui-ci doit appliquer préalablement sur la surface corporelle externe une couche de ladite composition. La couche de la composition étalée en amont peut sécher le temps que l'utilisateur applique l'appareil de massage sur la surface corporelle. Lorsque la composition a séché ou encore que les moyens de massage sont utilisés à sec sur la surface corporelle externe, les moyens massants peuvent rencontrer des difficultés à se déplacer sur la surface corporelle et ainsi causer une sensation d'inconfort.

Il existe également des applicateurs de composition cosmétique comportant un réservoir contenant la composition et une bille sphérique en acier ou en plastique logée dans une tête d'application laquelle est fixée sur le réservoir. Entre la bille et la tête d'application est prévu un jeu de manière que la bille puisse tourner librement sur elle-même lorsque celle-ci se déplace sur la surface corporelle externe et prélever une quantité de la composition dans la tête d'application en communication fluidique avec le réservoir pour l'étaler sur la surface corporelle externe. Un exemple d'applicateur est décrit dans le document US 4 940 350.

Toutefois, ce type d'applicateur ne permet pas de réaliser un massage de la surface corporelle suffisant pour permettre la pénétration de la composition dans la surface corporelle et augmenter l'action mécanique de la bille. D'autre part, un dépôt de la composition peut s'accumuler à l'endroit du jeu prévu entre la bille et la tête d'application ce qui peut empêcher la rotation de la bille. De plus, l'applicateur présente une surface d'application assez limitée du fait de la seule bille et de ce fait ne peut traiter que des petites zones de la surface corporelle externe.

Dans le document US 3 436 161 est décrit un applicateur de composition cosmétique comprenant une poignée portant un rouleau rotatif par rapport à celle-ci. Le rouleau comporte un revêtement externe déformable qui est muni de passages débouchant chacun dans un creux à la surface du revêtement. A l'intérieur du rouleau sont agencés un réservoir contenant la composition cosmétique et un dispositif de dosage permettant d'extraire des doses de la composition du réservoir et de les distribuer dans les creux de sorte que lors du déplacement du rouleau sur la surface corporelle d'un utilisateur, le revêtement se déforme au contact de la surface corporelle pour permettre aux doses de composition reçues dans les creux de s'étaler sur la surface corporelle.

Dans le document EP2735295A1 est décrit un appareil de massage tel que décrit dans le préambule de la revendication 1.

Toutefois, l'agencement de cet applicateur est complexe. Par ailleurs, un tel applicateur permet d'appliquer une dose/quantité de composition cosmétique sur la surface corporelle externe mais ne permet pas un massage de manière à permettre une pénétration de la composition dans la surface corporelle.

### 3. Objectifs de l'invention

L'invention a notamment pour objectif de pallier tout ou partie des inconvénients de l'art antérieur.

Un objectif de l'invention est de fournir un appareil de massage permettant de faciliter la pénétration d'une composition cosmétique à travers une surface corporelle d'un utilisateur en combinant l'action d'un massage et la distribution simultanée d'une composition cosmétique, dermatologique et/ou pharmaceutique sur la surface corporelle externe tout en étant simple et compact.

### 4. Résumé de l'invention

Ces objectifs sont atteints grâce à un appareil de massage tel que décrit dans la revendication 1.

Cette solution permet de résoudre les problèmes précités. En particulier, l'appareil de massage combine un massage de la surface corporelle externe avec une application/distribution de la composition cosmétique, dermatologique et/ou pharmaceutique sur la surface corporelle de l'utilisateur. L'application de la composition simultanément au massage de la surface corporelle permet de faciliter la pénétration de la composition, d'augmenter l'action mécanique, de faciliter le déplacement des moyens massants sur la peau du fait de la lubrification entre les moyens massants et la surface corporelle externe, et une distribution homogène de la composition sur la surface corporelle externe.

Selon une caractéristique de l'invention, les moyens massants sont amovibles de la tête de massage. Une telle configuration permet de remplacer facilement les moyens massants lorsque la composition cosmétique, dermatologique et/ou pharmaceutique que celui-ci comprend est épuisée.

Dans le même but, l'appareil de massage comprend un support fixé de manière amovible sur la tête de massage et sur lequel sont agencés les moyens massants.

Selon un exemple ne faisant pas partie de l'invention, les moyens massants comprennent une paroi creuse définissant une cavité interne agencée de sorte à contenir la composition cosmétique, dermatologique et/ou pharmaceutique.

Selon l'invention, l'appareil comprend un matériau poreux agencé dans la cavité des moyens massants et comprenant une porosité inférieure à celle de la paroi formée d'un matériau poreux. De la sorte, la composition est contenue dans les moyens massants et est mise en contact avec la surface corporelle via les pores de la paroi. Dans la paroi poreuse diffusante est aménagée alors une cavité. Cette cavité contient directement la composition à distribuer ou bien contenir un autre matériau poreux basse densité pour maximiser la quantité de composition embarquée, ainsi que de maîtriser le débit diffusé.

Selon un exemple ne faisant pas partie de l'invention, les moyens massants comprennent une paroi recouverte de la composition cosmétique, dermatologique et/ou pharmaceutique. De la sorte la composition est en contact directement avec la surface corporelle de l'utilisateur.

Selon encore une autre caractéristique de l'invention, les moyens massants comprennent une paroi recouverte d'un revêtement en matériau textile comportant des microcapsules lesquelles sont agencées de sorte à contenir la composition cosmétique, dermatologique et/ou pharmaceutique. Cette configuration permet une libération progressive de la composition par casse des micro capsules, tout en maîtrisant la douceur du massage par le textile.

Selon un exemple ne faisant pas partie de l'invention, l'appareil de massage comprend un réservoir agencé de sorte à contenir la composition cosmétique et/ou dermatologique et/ou pharmaceutique. Cette configuration permet de faciliter le conditionnement de la composition.

Selon un exemple ne faisant pas partie de l'invention, le réservoir est formé par une enveloppe présentant une paroi souple et déformable pourvue d'une fente, la paroi étant agencée de manière à épouser une surface interne de la cavité des moyens massants. Le moyen de massage est alors souple et conformable aux contours du visage.

Selon un exemple ne faisant pas partie de l'invention, l'appareil comprend un mécanisme d'extraction configuré de manière à agir sur le réservoir pour extraire un contenu.

Selon une autre caractéristique de l'invention, la paroi des moyens massants est sphérique, semi-sphérique ou cylindrique selon le type de massage à reproduire.

Selon une autre caractéristique, les moyens massants sont montés mobiles suivant au moins un axe de rotation. Cette configuration permet d'augmenter également l'action mécanique des moyens massants sur la surface corporelle.

Selon un exemple ne faisant pas partie de l'invention, l'appareil comprend la composition cosmétique, dermatologique et/ou pharmaceutique, ce qui permet d'augmenter le volume de composition embarqué.

### 5. Liste des figures

D'autres caractéristiques et avantages innovants ressortiront de la description ci-après, fournie à titre indicatif et nullement limitatif, en référence aux dessins annexés, dans lesquels :
- La figure 1 est une coupe schématique d'un appareil de massage ne faisant pas partie de l'invention;
- Les figures 2, 4 et 5 sont des vues en coupe schématique de différents exemples de réalisation d'un moyen massant d'un appareil de massage un exemple ne faisant pas partie de l'invention ;
- La figure 3 illustre une vue en perspective d'un exemple de réalisation d'un moyen massant d'un appareil de massage selon l'invention ;
- La figure 6 est une vue en perspective et partielle de la tête de massage de l'appareil de massage selon la figure 1 ;
- Les figures 7 et 8 illustrent, selon des vues éclatées, une alternative du mode de réalisation illustré sur la figure 6.
- La figure 9 représente une coupe schématique de la tête de massage selon l'invention ; et,
- La figure 10 représente une vue en coupe schématique d'un autre mode de réalisation d'un appareil de massage un exemple ne faisant pas partie de l'invention.
- Les figures 11a et 11b représentent une alternative de l'appareil de massage tel que illustré à la figure 10 ne faisant pas partie de l'invention.

### 6. Description détaillée

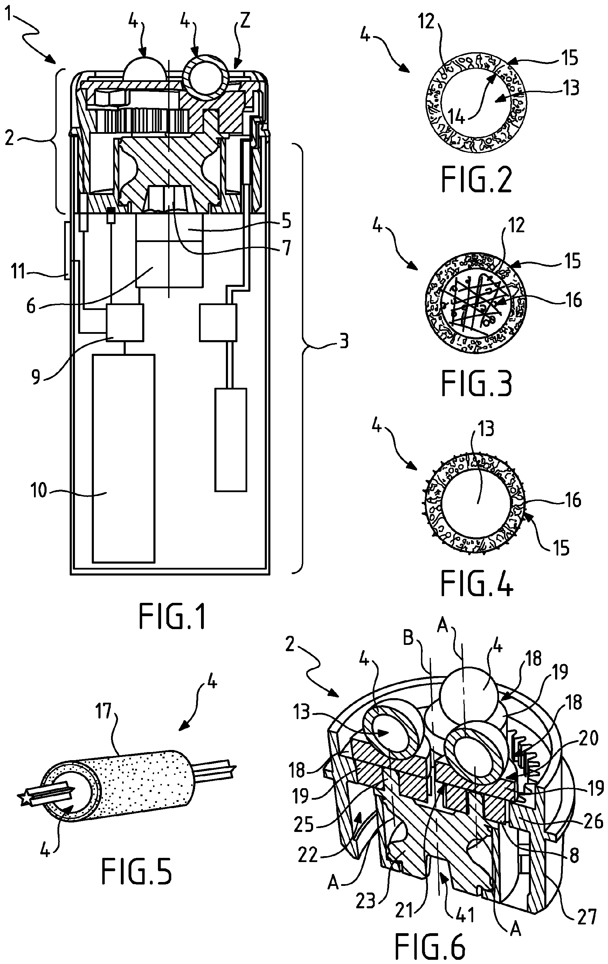

Sur la figure 1 est illustré un appareil de massage 1. L'appareil de massage 1 comprend une tête de massage 2 et un boîtier 3 portant la tête de massage 2. La tête de massage 2 est montée de manière amovible sur le boîtier. La tête de massage 2 comporte des moyens massants 4 mobiles par rapport à la tête de massage 2. Ces moyens massants 4 sont agencés de sorte à être en contact avec une surface corporelle externe d'un utilisateur. Préférentiellement, mais non limitativement, les moyens massants 4 sont conçus pour exercer une action mécanique sur une surface corporelle externe, telle que la peau du visage d'un utilisateur.

Pour cela, le boîtier 3 comprend des moyens d'entrainement 5 des moyens massants 4. Les moyens d'entrainement 5 sont actionnés, ici, par un moteur électrique 6 lequel est logé à l'intérieur du boitier 3 et transmettent le mouvement du moteur électrique 6 aux moyens massants 4. Plus précisément, les moyens d'entrainement 5 comprennent un réducteur (non représenté) entrainant un arbre de sortie 7 agencé à une des extrémités du boitier 3 se connectant à la tête de massage 2.

Le moteur électrique 6 est piloté par une unité de commande 9 alimentée par un moyen d'alimentation logé à l'intérieur du boîtier 3. Le moyen d'alimentation est ici un bloc de batterie 10. Bien entendu, le moyen d'alimentation pourrait être un adaptateur destiné à être branché sur un réseau électrique. L'unité de commande 9 est en outre raccordée à une interface de commande manuelle 11 accessible depuis l'extérieur du boitier. L'interface de commande manuelle 11 peut par exemple comprendre un interrupteur marche-arrêt et/ou des moyens de sélection manuelle de programmes de fonctionnement.

Selon l'invention, les moyens massants 4 sont conçus de manière à travailler/masser la surface corporelle externe et à appliquer une composition cosmétique, dermatologique et/ou pharmaceutique simultanément. La composition cosmétique, dermatologique et/ou pharmaceutique présente des propriétés hydratantes, anti-rides, anti-fatigue, anti-cernes, anti-âge, anti-acné, anti-cellulite, permettant de donner un coup d'éclat, de remodelage, exfoliantes, etc. La composition se présente sous une forme plus ou moins liquide, pâteuse, ou solide à température ambiante.

Selon une caractéristique de l'invention, les moyens massants 4 sont agencés de sorte à pouvoir comprendre la composition cosmétique, dermatologique et/ou pharmaceutique de manière, lors d'une utilisation, à appliquer ladite composition sur la surface corporelle externe et masser ladite surface corporelle externe simultanément. Dans la présente description, nous comprenons par le terme « comprendre » dans l'expression « les moyens massants sont agencés de sorte à comprendre... » le fait que les moyens massants sont chargés ou stockent la composition cosmétique, dermatologique et/ou pharmaceutique. En d'autres termes, les moyens massants 4 sont déjà approvisionnés en composition avant leur actionnement et avant que ceux-ci ne soient en contact avec la surface corporelle de l'utilisateur.

A cet effet, dans un premier exemple ne faisant pas partie de l'invention, des moyens massants tel qu'illustré sur la figure 2, les moyens massants 4 comprennent une paroi 12 creuse définissant une cavité 13 interne. Cette dernière est délimitée par une surface interne 14 de la paroi 12 laquelle est opposée à une surface externe 15 destinée à être en contact avec la surface corporelle. La cavité 13 interne est agencée de sorte à contenir la composition cosmétique, dermatologique et/ou pharmaceutique. La paroi creuse 12 est alors poreuse de manière à permettre la diffusion de la composition disposée dans la cavité 13 interne vers la surface corporelle. Nous entendons dans la présente description par le terme «poreux», une pluralité de pores ou d'ouvertures traversant la paroi de part et d'autre de la surface interne et externe de manière à établir une communication fluidique entre la cavité 13 interne de chaque moyen massant 4 et l'extérieur de celui-ci. La paroi 12 poreuse est ici réalisée dans un matériau polymère ou organique. Le matériau est de préférence, mais non limitativement, choisi dans le groupe feutres - la matière peut être naturelle (poils d'animaux, sphaigne, coton, silice) ou de synthèse (PE/PET, mousse d'alumine). La paroi 12 des moyens massants 4 présente une épaisseur comprise entre 0.5 et 1.5 mm de sorte à favoriser une circulation rapide de la composition entre la cavité 13 interne et la surface externe 15 des moyens massants 4. Dans ce mode de réalisation, la composition présente une forme liquide ou plus ou moins liquide de sorte que son pompage par la paroi 12 soit contrôlé en fonction de la viscosité et la tension de surface de la composition.

Suivant un mode de réalisation selon l'invention, des moyens massants 4 susmentionné et illustré sur la figure 3, un matériau absorbant est agencé dans la cavité 13 interne de chaque moyen de massage. Plus précisément, ce matériau absorbant comprend, de préférence, mais non limitativement, un matériau poreux 16 permettant de stocker la composition à l'intérieur de la cavité 13 interne. Le matériau poreux 16 est choisi parmi le PE/PET, laine de roche, sphaigne, coton, feutre. Le matériau poreux 16 présente une porosité entre 30 et 70%, idéalement 50%. Le matériau diffusant 12 est plutôt à sélectionner parmi feutre, mousse d'alumine ou silice, et dur (dureté > 70 Shore A). La composition se présente alors sous une forme plus ou moins liquide et sa viscosité est comprise entre 0,5 cP et 100 cP, de préférence 50 cP.

Suivant un autre exemple ne faisant pas partie de l'invention, des moyens massants 4 illustré sur la figure 4, la paroi 12 du moyen massant 4 est agencée de sorte à être recouverte de la composition cosmétique, dermatologique et/ou pharmaceutique. Plus précisément, la surface externe 15 de la paroi est composée de cires, huiles et graisses, solides à température ambiante. La dureté de la surface externe 15 est alors comprise entre 30 et 120 Shore A, préférentiellement 80 Shore A. Ainsi, lorsque les moyens massants 4 travaillent la surface corporelle, la composition qui est disposée sur la surface externe de la paroi 12, est directement en contact avec la surface corporelle et se dépose par érosion/frottement sur celle-ci afin de l'enduire de la composition cosmétique, dermatologique et/ou pharmaceutique. Dans ce mode de réalisation, la composition présente préférentiellement, mais non limitativement, une forme solide. La composition adhère mieux aux moyens massants correspondant et permet de conserver son intégrité. Le dépôt de la composition est également plus facile. La composition cosmétique, dermatologique et/ou cosmétique se présente également sous la forme d'une couche d'épaisseur comprise entre 0.5 et 5 mm.

Suivant une alternative de cet exemple illustré sur la figure 4, la paroi 12 de chaque moyen massant 14 est recouverte d'un revêtement 17 imprégné de la composition cosmétique, dermatologique et/ou pharmaceutique. Dans le cas présent, le revêtement 17 est un matériau textile comportant des microcapsules contenant la composition. Bien entendu, le revêtement 17 peut être choisi dans le groupe comprenant les mousses, les éponges et autres matériaux absorbants. Le matériau textile peut être tissé ou non tissé. La composition cosmétique, dermatologique et/ou pharmaceutique se dépose par frottement du matériau textile lorsque les moyens massants 4 se déplacent sur/travaillent la surface corporelle externe de l'utilisateur.

Le travail de la surface corporelle est réalisé par déplacement des moyens massants 4 contre la surface corporelle. Le déplacement des moyens massants réalise un pétrissage de la surface corporelle voire un pincement de celle-ci.

Suivant un exemple ne faisant pas partie de l'invention, illustré sur les figures 1 et 6, les moyens massants 4 sont montés sur la tête de massage 2 de manière amovible afin de les remplacer lorsque la composition cosmétique, dermatologique et/ou pharmaceutique à appliquer est épuisée. Ici, les moyens massants 4 présentent une paroi sphérique ou semi-sphérique. En particulier, le moyens massants 4 comprennent trois billes qui sont mobiles chacune en rotation dans la tête de massage suivant un axe de rotation A parallèle l'un à l'autre. Plus précisément, les billes sont montées chacune sur une tête de travail 18 laquelle comporte un disque 19 qui est centré par rapport à l'axe de rotation A. Le disque 19 présente une face supérieure 20 sur laquelle la bille correspondante est liée de manière rigide au disque 19. Chaque tête de travail 18 comprend en outre un pignon planétaire 8 lié de manière rigide à une face inférieure 21 du disque 19 correspondant, opposée à la face supérieure 20, et qui est coaxial à l'axe de rotation A. La tête de massage comprend des moyens de manœuvre 22 coopérant d'une part avec les moyens d'entrainement 5, et d'autre part avec le pignon planétaire 20. En particulier, les moyens de manœuvre 22 comprennent un arbre de manœuvre 23 d'axe B qui comprend sur une surface inférieure un logement de réception 41 de l'arbre de sortie et sur une surface supérieure des pions 25 engagés chacun dans un alésage axial d'un pignon planétaire 8. L'axe de rotation A de chaque bille est décalé par rapport à l'axe de l'arbre de manœuvre B. Les moyens de manœuvre 22 comprennent en outre une couronne périphérique 26 fixe liée de manière rigide à un carter 27 constitutif de la tête de massage. Chacun des pignons planétaires 8 engrène la couronne périphérique 26 fixe de sorte que la rotation de l'arbre de manœuvre 23 entraîne le mouvement des billes. Les billes présentent un diamètre compris entre 5 et 15 mm. De préférence, mais non limitativement, le diamètre de chaque bille est de 10 mm. Les billes sont chargées de la composition cosmétique, dermatologique et/ou pharmaceutique selon les modes de réalisation illustrés sur les figures 2 à 5.

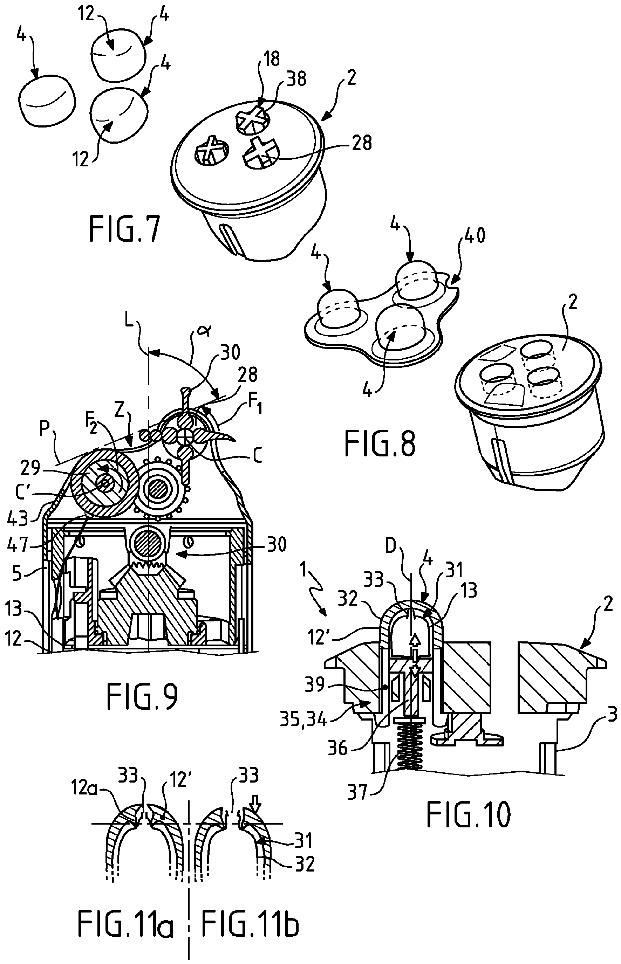

Selon une alternative à cet exemple illustré sur la figure 7, chaque tête de travail 18 comprend une embase 38 dont la section présente une forme en croix. La paroi 12 des moyens massants 4 est alors semi-shérique et présente une zone en creux femelle de manière à recevoir de manière amovible l'embase 38 mâle par emboîtement ou clipsage.

Selon encore une autre alternative, ne faisant pas partie de l'invention, illustrée sur la figure 8, les moyens massants 4 sont montés sur un support 40 lequel est fixé de manière amovible sur la tête de massage 2. Dans ce cas, le support 40 permet un remplacement de celui-ci et des moyens massants 4 lorsque la composition est épuisée. Le remplacement est également possible en cas de détérioration du support ou des moyens massants. Chaque moyen massant 4 sous forme de bille peut être monté sur un axe en plastique qui est lui-même libre en rotation. L'axe est par exemple orthogonal au plan du support 40. Ainsi, les moyens massants 4 sont libres en rotation pendant l'utilisation.

Suivant un autre exemple ne faisant pas partie de l'invention, illustré sur la figure 9, les moyens massants 4 sont montés sur la tête de massage 2 de manière amovible afin de les remplacer lorsque la composition cosmétique, dermatologique et/ou pharmaceutique à appliquer est épuisée. Les moyens massants présentent chacun une paroi cylindrique. En particulier, les moyens massants 4 illustrés comprennent deux rouleaux cylindriques qui sont mobiles chacun en rotation sur eux même autour d'un axe de rotation C, C' . Les axes C, C' de rotation sont parallèles l'un à l'autre. Les deux rouleaux de massage dénommés ci-après premier rouleau 28 et deuxième rouleau 29 sont distants l'un de l'autre et sont séparés par une zone de travail Z que l'on peut envisager ajustable ou variable dans une variante de construction. Selon une alternative de ce mode de réalisation permettant de réaliser un pincement d'une portion de la surface corporelle, le premier rouleau 28 de massage comprend au moins une palette 30 qui s'étend radialement en saillie depuis la surface du premier rouleau 28. De manière préférée, mais non limitative, le premier rouleau 28 est pourvu de quatre palettes 30 réparties régulièrement à la périphérie du premier rouleau. Selon cette alternative, le premier rouleau 28 et le deuxième rouleau 29 de massage sont agencés au sein de la tête de massage de manière qu'un plan P tangent au premier rouleau 28 et au deuxième rouleau 29 et situé vers l'extérieur de la tête de massage forme avec un axe du boitier d'entraînement un angle non nul et différent de l'angle droit. Le premier rouleau et/ou le deuxième rouleau sont chargés de la composition cosmétique, dermatologique et/ou pharmaceutique selon les modes de réalisation illustrés sur les figures 2 à 5. Les palettes 30 peuvent être également revêtues d'une couche de la composition cosmétique et/ou dermatologique et/ou pharmaceutique.

Suivant un autre exemple ne faisant pas partie de l'invention illustré sur la figure 10, les moyens massants 4 sont montés de manière amovible dans la tête de massage. Ceux-ci présentent ici une paroi 12' semi-sphérique. En particulier, les moyens massants 4 comprennent des demi-sphères ou demi-billes qui sont mobiles en translation dans la tête de massage 2 suivant un axe D ici vertical en référence à la figure 10. L'appareil de massage 1 comprend un réservoir 31 agencé de sorte à contenir la composition cosmétique, dermatologique et/ou pharmaceutique. En particulier, le réservoir 31 est disposé dans la cavité 13 interne de la paroi 12' des moyens massants 4. Ce réservoir 13 est formé par une enveloppe présentant une paroi souple 32 et déformable. Cette dernière épouse la surface interne de la cavité 13 interne des moyens massants 4. La paroi souple 32 comprend une fente 33 permettant la sortie de la composition du réservoir vers la paroi 12' des moyens massants 4 qui est poreuse dans le présent mode de réalisation mais peut également ne pas l'être. Selon une caractéristique de ce mode de réalisation, l'appareil de massage comprend un mécanisme d'extraction 34 configuré de manière à agir sur le réservoir 31 pour extraire son contenu. Le mécanisme d'extraction 34 comprend une pompe 35 coopérant avec le réservoir 31. En particulier, la pompe 35 est installée dans la tête de massage et en avant du réservoir 31 placé dans la cavité interne de chaque moyen massant 4. La pompe 35 comprend un corps cylindrique 39 et un piston 36 soumis à l'action d'un ressort 37 agencé dans le boîtier 3. Le corps cylindrique 39 du piston est relié par clipsage au moyen massant 4 correspondant. Un moyen de pression est mis en contact avec le piston 36 permettant de mettre le réservoir 31 sous pression. Lors de l'application, la peau appuie sur la paroi 12' ce qui autorise l'ouverture de la fente 33 et la diffusion de la composition.

Alternativement à l'exemple ne faisant pas partie de l'invention, illustré à la figure 10 et tel que visible aux figure 11a et 11b, la paroi 12' des moyens massants n'est pas constituée d'un matériau poreux mais présente une ouverture adjacente à la fente 33 du réservoir. Quand l'appareil est en état libre sans pression contre la peau, la fente 33 du réservoir 31 reste fermée. La paroi 12' présente en outre des moyens d'appui 12a permettant l'ouverture de la fente 33 tel que visible à la figure 11b.

Nous allons maintenant décrire le fonctionnement de l'appareil de massage. L'utilisateur applique la tête de massage de l'appareil de massage 1 contre une surface corporelle externe à travailler puis l'utilisateur met l'appareil sous tension au moyen de l'interface de commande manuelle 11. Les moyens massants 4, en l'occurrence les billes ou les rouleaux sont actionnés en rotation par les moyens d'entrainement via le moteur électrique 6. La rotation des billes ou des rouleaux permet de masser la surface corporelle externe de l'utilisateur. Simultanément à ce massage, la composition cosmétique que comprennent les moyens massants 4 est disposée sur la surface corporelle externe soit par relargage depuis la cavité 13 interne soit par frottement direct avec la surface corporelle externe de l'utilisateur. Le choix des textures, viscosités et formes de distribution de la composition peut ainsi être parfaitement adapté au massage (stimuli mécaniques) choisi. Notamment, cela permet d'ajuster le massage et le temps de pénétration de la composition dans la peau. Cela permet aussi d'apposer le cosmétique avec le massage sélectionné pour plus d'efficacité et de sécurité. Ainsi le déplacement des moyens massants 4 est facilité puisque ceux-ci sont lubrifiés et la pénétration de la composition cosmétique, dermatologique et/ou pharmaceutique améliorée.

L'invention est décrite dans ce qui précède à titre d'exemple. Il est entendu que la personne de l'art est à même de réaliser différentes variantes de réalisation de l'invention telle que décrite dans les revendications, en associant par exemple les différentes caractéristiques ci-dessus prises seules ou en combinaison, sans pour autant sortir du cadre de l'invention telle que décrite dans les revendications.

## Revendications

1. Appareil de massage (1) comprenant:
- une tête de massage (2) comportant des moyens massants (4) mobiles en rotation par rapport à la tête de massage (2) et qui sont agencés de sorte à être en contact avec une surface corporelle externe d'un utilisateur ; et,
- un boitier (3) portant la tête de massage (2) et comportant des moyens d'entraînement actionnant les moyens massants (4) ; où au moins un des moyens massants (4) est chargé de ou stocke une composition cosmétique, dermatologique et/ou pharmaceutique de manière à, lors d'une utilisation, appliquer ladite composition sur la surface corporelle externe et masser ladite surface corporelle externe simultanément, les moyens massants (4) étant déjà approvisionnés en composition avant leur actionnement et avant que ceux-ci ne soient en contact avec la surface corporelle de l'utilisateur et où les moyens massants (4) comprennent une paroi (12) creuse définissant une cavité (13) interne agencée de sorte à contenir la composition cosmétique, dermatologique et/ou pharmaceutique, **caractérisé par** un matériau poreux (16) étant agencé dans la cavité (13) des moyens massants (4) et comprenant une porosité inférieure à celle de la paroi (12) formée d'un matériau poreux.

2. Appareil (1) selon la revendication 1, **caractérisé en ce que** les moyens massants sont amovibles de la tête de massage.

3. Appareil (1) selon la revendication 2, **caractérisé en ce qu'**il comprend un support (40) fixé de manière amovible sur la tête de massage (2) et sur lequel sont agencés les moyens massants (4).

4. Appareil (1) selon l'une quelconque des revendications 1 à 3, **caractérisé en ce que** la paroi des moyens (4) massants est sphérique, semi-sphérique ou cylindrique.

5. Appareil (1) selon l'une quelconque des revendications 1 à 4, **caractérisé en ce que** les moyens massants (4) sont montés mobiles suivant au moins un axe de rotation.

## Patentansprüche

1. Massagegerät (1), umfassend:
- einen Massagekopf (2), der massierende Mittel (4) umfasst, die in Drehung in Bezug auf den Massagekopf (2) beweglich sind, und die derart eingerichtet sind, dass sie mit einer äußeren Körperoberfläche eines Benutzers in Kontakt sind; und
- ein Gehäuse (3), das den Massagekopf (2) trägt und Antriebsmittel umfasst, die die massierenden Mittel (4) betätigen;
wobei mindestens eines der massierenden Mittel (4) mit einer kosmetischen, dermatologischen und/oder pharmazeutischen Zusammensetzung beladen ist oder diese derart lagert, dass bei einer Verwendung die Zusammensetzung auf die externe Körperoberfläche aufgetragen und gleichzeitig die externe Oberfläche massiert wird, wobei die massierenden Mittel (4) vor ihrem Betätigen und bevor sie mit der Körperoberfläche des Benutzers in Kontakt sind, bereits geliefert sind, und wobei die massierenden Mittel (4) eine interne Hohlwand (12), die einen Hohlraum (13) definiert, umfassen, der derart eingerichtet ist, dass er die kosmetische, dermatologische und/oder pharmazeutische Zusammensetzung enthält, **gekennzeichnet durch** ein poriges Material (16), das in dem Hohlraum (13) der massierenden Mittel (4) eingerichtet ist und eine Porosität umfasst, die geringer ist als die der Wand (12), die aus einem porigen Material gebildet ist.

2. Gerät (1) nach Anspruch 1, **dadurch gekennzeichnet, dass** die massierenden Mittel von dem Massagekopf abnehmbar sind.

3. Gerät (1) nach Anspruch 2, **dadurch gekennzeichnet, dass** es einen Träger (40) umfasst, der abnehmbar auf dem Massagekopf (2) befestigt ist, und auf dem die massierenden Mittel (4) eingerichtet sind

4. Gerät (1) nach einem der Ansprüche 1 bis 3, **dadurch gekennzeichnet, dass** die Wand der massierenden Mittel (4) kugelförmig, halbkugelförmig oder zylindrisch ist.

5. Gerät (1) nach einem der Ansprüche 1 bis 4, **dadurch gekennzeichnet, dass** die massierenden Mittel (4) entlang mindestens einer Rotationsachse beweglich montiert sind.

## Claims

1. Massage device (1) comprising:
- a massage head (2) including massaging means (4) mobile in rotation with respect to the massage head (2) and which are arranged so as to be in contact with an external body surface of a user; and,
- a casing (3) carrying the massage head (2) and including drive means actuating the massaging means (4);
where at least one of the massaging means (4) is filled with or stores a cosmetic, dermatological and/or pharmaceutical composition, so as to, during a use, apply said composition on the external body surface and massage said external body surface simultaneously, the massaging means (4) already being provided in composition before the actuation thereof and before these are in contact with the body surface of the user and where the massaging means (4) comprise a hollow wall (12) defining an internal cavity (13) arranged so as to contain the cosmetic, dermatological and/or pharmaceutical composition, **characterised by** a porous material (16) being arranged in the cavity (13) of the massaging means (4) and comprising a porosity less than that of the wall (12) formed of a porous material.

2. Device (1) according to claim 1, **characterised in that** the massaging means are removable from the massage head.

3. Device (1) according to claim 2, **characterised in that** it comprises a support (40) removably fixed on the massage head (2) and on which the massaging means (4) are arranged.

4. Device (1) according to any one of claims 1 to 3, **characterised in that** the wall of the massaging means (4) is spherical, semi-spherical or cylindrical.

5. Device (1) according to any one of claims 1 to 4, **characterised in that** the massaging means (4) are mounted mobile about at least one axis of rotation.
